# EUROPÄISCHE PATENTSCHRIFT

(11) **EP 4 306 147 B1**
(45) Veröffentlichungstag und Bekanntmachung des Hinweises auf die Patenterteilung: **14.10.2026**
(21) Anmeldenummer: 23185072.8
(22) Anmeldetag: 12.07.2023
(51) Int. Cl.: A61M 5/145, A61M 5/168

(54) **SPRITZENPUMPE MIT KOLBENBREMSE**
SYRINGE PUMP WITH PISTON BRAKE
POMPE À SERINGUE AVEC FREIN À PISTON

(30) Priorität: 14.07.2022 DE 102022117611
(43) Veröffentlichungstag der Anmeldung: 17.01.2024
(73) Patentinhaber: B. Braun Melsungen AG, 34212 Melsungen (DE)
(72) Erfinder: ALJETS, Dirk, 34466 Wolfhagen (DE)
(74) Vertreter: Winter, Brandl - Partnerschaft mbB

(56) Entgegenhaltungen:
- DE-U1- 20 200 885
- DE-U1- 29 812 065
- US-A1- 2002 043 951
- US-A1- 2004 057 855

## Beschreibung

Die vorliegende Offenbarung betrifft eine als Spritzenpumpe ausgebildete Infusionspumpe gemäß dem Oberbegriff des Patentanspruchs 1.

### Hintergrund der Offenbarung

In derartige Spritzenpumpen werden auswechselbare Spritzen eingesetzt, deren jeweiliger Spritzenkolben über eine jeweilige Kolbenstange der Spritzenpumpe automatisch und einstellbar bzw. programmierbar in den jeweiligen Spritzenzylinder eingeschoben werden kann. Auf diese Weise wird die jeweilige medizinische Flüssigkeit innerhalb des Spritzenzylinders automatisch und einstellbar bzw. programmierbar aus dem jeweiligen Spritzenzylinder herausgedrückt bzw. herausgefördert. Dazu benötigt die Spritzenpumpe eine entlang einer Spritzenlängsachse linearbewegliche Baugruppe.

### Stand der Technik

Die Druckschrift US 2004/0057855 A1 der Anmelderin offenbart eine Spritzenpumpe mit einer federgespannten Kolbenbremse.

Die Druckschrift US 2002/0043951 A1 offenbart eine Spritzenpumpe, bei welcher der elektrische Strom des Pumpenmotors überwacht wird.

Die Druckschrift EP 0 314 880 A2 offenbart eine Spritzenpumpe, bei welcher die linearbewegliche Baugruppe als Schieber bezeichnet wird. In dem Schieber ist ein (einziger) Schalter angeordnet, der mit einem Ausrastdetektor, mit einem Drucksensor und mit einem Spritzendetektor in Wirkverbindung ist.
- Der Drucksensor schaltet bei Erreichen oder Überschreiten eines Grenzwerts einer Betätigungskraft in Einschubrichtung des Spritzenkolbens.
- Der Spritzendetektor erkennt das lagerichtige Einsetzen der Spritze und erzeugt ein Signal zur Inbetriebsetzung des Antriebs nur dann, wenn die Spritze so eingesetzt ist, dass sie - entlang der Spritzenlängsachse betrachtet - an beidseitigen Spritzenhaltern abgestützt ist.

Die Druckschrift EP 2 606 922 B1 offenbart eine Spritzenpumpe, wobei die linearbewegliche Baugruppe als Antriebskopf bezeichnet wird. Ein korrektes Anliegen einer mit dem Kolben verbundenen Kolbenplatte der Spritze am Antriebskopf wird mit einem Kraftsensor bestimmt. Mittels eines Potentiometers (an einem Klemmbügel) wird eine Spritzengröße, genauer gesagt der Durchmesser des Spritzenzylinders gemessen.

Die Druckschriften DE 202 00 855 U1 und EP 1 329 232 B1 der Anmelderin offenbaren eine Spritzenpumpe, bei der über einen Sensor der Durchmesser des Spritzenzylinders bzw. die Spritzengröße ermittelt wird. Eine solche Spritze hat dabei in der Regel eine Kolbenplatte am freien äußeren Ende des Spritzenkolbens bzw. der Kolbenstange, um über diese eine Druckkraft auf den Spritzenkolben ausüben zu können. Ein korrektes Anliegen der Kolbenplatte an einem Antriebskopf der linearbeweglichen Baugruppe wird daher mit einem Kolbenplattensensor bestimmt, der auf die Anwesenheit der Kolbenplatte reagiert und dadurch erkennt, dass der Antriebskopf an die Kolbenplatte einer eingelegten Spritze herangefahren ist. Des Weiteren ist eine Kolbenbremse in der bekannten Spritzenpumpe vorgesehen. Diese Kolbenbremse hat ein als eine Art Klinge ausgebildetes Brems-/Blockierelement, das über einen Elektromotor radial zur Kolbenstange in Richtung zur Kolbenstange bewegt werden kann und in diese (formschlüssig) eingreift. Damit kann die Kolbenstange axial festgelegt werden, wodurch eine ungewollte Bewegung des Kolbens relativ zum Spritzenzylinder verhindert wird. Zwischen der Kolbenstange und einer dazu parallelen Antriebsstange der Spritzenpumpe bzw. deren linearbeweglichen Baugruppe ist ein Stützlager angeordnet, an dem sich die Kolbenplatte beim Eingriff des Bremselements radial abstützen kann.

Eine vorbeschriebene Kolbenbremse ist auch in der Druckschrift DE 20 2021 103 509 U1 der Anmelderin offenbart.

### Kurzbeschreibung der Offenbarung

Aufgabe der vorliegenden Offenbarung ist es, bei einer als Spritzenpumpe ausgebildeten medizinischen Infusionspumpe, die eine Kolbenbremse der vorstehenden Bauart aufweist, die Spritzenüberwachung, insbesondere die Kolbenüberwachung zu optimieren.

Diese Aufgabe wird durch eine Spritzenpumpe mit den Merkmalen des Anspruchs 1 gelöst. Weitere vorteilhafte Ausgestaltungen sind Gegenstand der Unteransprüche.

Der Grundgedanke der vorliegenden Offenbarung beruht im Wesentlichen auf der Integration von Sensorik in der Kolbenbremse zur vorrichtungstechnischen Vereinfachung oder zur Verbesserung der Spritzenüberwachung. Mit der Kolbenbremse wird die Kolbenstange und damit der Kolben dabei so lange festgelegt, bis die erfindungsgemäße Sensorik zulässige Erfassungs-Ergebnisse (beispielsweise bezüglich korrekter Positionierung der Spritze und/oder korrekter Funktion der Spritze selbst und/oder korrekte Spritzengröße, etc.) liefert und daher die Spritzenpumpe für den Normalbetrieb bereit ist. Bei unzulässigen Ergebnissen muss die Spritze wieder aus der Spritzenpumpe entnommen und entweder korrekt montiert oder korrekt eingesetzt oder ausgetauscht werden (ansonsten kann die Spritzenpumpe die eingelegte Spritze nicht betätigen).

Mit der offenbarungsgemäßen Sensorik erfolgt eine Erkennung und/oder Bewertung der eingesetzten Spritze in Wirkverbindung mit dem Antrieb der Kolbenbremse. Diese Erkennung und/oder Bewertung kann durch die Integration der Sensorik in die Kolbenbremse den vorrichtungstechnischen Aufwand der Spritzenpumpe verringern, oder die Erkennung und/oder Bewertung kann zusätzlich zur Erkennung und/oder Bewertung einer weiteren Sensorik erfolgen, z.B. zusätzlich zu der aus der Druckschrift EP 1 329 232 B1 bekannten Sensorik.

Die beanspruchte Spritzenpumpe hat demzufolge einen Aufnahmebereich für eine Spritze, in den hinein ein Antriebskopf linear bewegbar ist. Mindestens ein Bremselement einer Kolbenbremse der Spritzenpumpe ist im Wesentlichen radial oder mit einer radialen Bewegungsrichtungskomponente (also schräg) zu einer Kolbenstange der Spritze bewegbar, um die Kolbenstange kraftschlüssig (Reibkraftbremse) und/oder formschlüssig (z.B. mit einem als Klinge ausgebildeten Bremselement) festzulegen. Offenbarungsgemäß ist durch eine in einen Antrieb der Kolbenbremse integrierte Krafterfassungsvorrichtung und/oder Wegerfassungsvorrichtung eine Anwesenheit und/oder eine (korrekte) Position und/oder ein (korrekter) Durchmesser der Kolbenstange der Spritze ermittelbar. In anderen Worten ausgedrückt, wenn die Krafterfassungsvorrichtung bzw. Wegerfassungsvorrichtung ein (z.B. gegenüber einem abgespeicherten Wert) unzulässiges Ergebnis liefert, wird vorzugsweise ein Signal, insbesondere ein Alarmsignal oder ein Hinweis im Display erzeugt, damit die Spritze wieder aus der Spritzenpumpe entnommen und entweder korrekt montiert oder korrekt eingesetzt oder ausgetauscht werden muss. Außerdem kann die Funktion der Pumpe temporär gestoppt werden.

Bei einer bevorzugten Ausgestaltung der Spritzenpumpe weist der Antrieb (der Kolbenstangen-Bremsvorrichtung) einen Elektromotor, ein Piezoantrieb, einen Hydraulikmotor oder einen Pneumatikmotor auf, mit dem das mindestens eine Bremselement (der Kolbenstangen-Bremsvorrichtung) bewegbar ist.

Bei einer Weiterbildung des Prinzips "Krafterfassung" ist ein Kraftanstieg erfassbar, und es wird erfasst, ob der Kraftanstieg, z.B. Steigung, unterhalb eines Grenzwertes ist. In diesem Fall ist die Kolbenstange beschädigt oder falsch montiert. Damit ist die gesamte Spritze nicht korrekt gewählt, insbesondere ist sie defekt, oder die Kolbenstange ist nicht korrekt an den Kolben montiert.

Die Krafterfassungsvorrichtung kann eine Drehmomenterfassungsvorrichtung sein, da ein Drehmoment des betroffenen Bauteils des Abtriebs schließlich auch in eine Kraft des mindestens einen Bremselements umgewandelt wird.

Vorrichtungstechnisch einfach ist es, wenn die Drehmomenterfassungsvorrichtung den Motorstrom des Elektromotors (oder der Hydraulik-/Pneumatikdruck) erfasst. Dies gilt insbesondere, wenn auch aus anderen Gründen eine Motorstromerfassung nötig ist.

Besonders sicher ist die Kolbenstangenerkennung, wenn ein Motorstromanstieg erfassbar ist, und wenn erfasst wird, ob dieser Motorstromanstieg unterhalb eines Grenzwertes ist. Dies gilt insbesondere, wenn auch aus anderen Gründen eine Motorstromerfassung nötig ist, z.B. um den Motorstrom abzuschalten. Wenn der Motorstromanstieg unterhalb des Grenzwertes bleibt, ist die Kolbenstange beschädigt, oder falsch montiert. Damit ist die gesamte Spritze nicht korrekt, insbesondere defekt, oder die Kolbenstange ist nicht korrekt an den Kolben montiert.

Die Wegerfassung kann konkret mittels einer Umdrehungserfassungsvorrichtung erfolgen, die mit einem rotierenden Bauteil des Antriebs in Wirkverbindung ist. Da eine Rotation des betroffenen Bauteils des Abtriebs schließlich auch in einen Weg des mindestens einen Bremselements umgewandelt wird.

Bei der Weiterbildung mit dem Elektromotor kann das rotierende Bauteil ein Rotor, insbesondere eine Motorwelle oder ein Gehäuse des Elektromotors sein.

Die Wegerfassung kann mittels eines rotatorischen oder linearen Potentiometers erfolgen.

Es können auch mehrere Bremselemente am Außenumfang der Kolbenstange verteilt sein, die entsprechend von mehreren Seiten gleichzeitig und/oder synchron an die Kolbenstange heranbewegt werden können. Vorzugsweise sind diese mehreren Bremselemente (z.B. nach dem Prinzip einer Fotoblende) gleichmäßig am Umfang der Kolbenstange verteilt. Damit kann eine Kolbenbremse gebildet sein, die die Kolbenstange besonders sicher hält, und die keine Querkraft in die Kolbenstange einleitet, wenn diese konzentrisch zur (erwarteten) Spritzenlängsachse ausgerichtet ist.

Ergänzend zur genannten Krafterfassungsvorrichtung und/oder Wegerfassungsvorrichtung kann auch ein (weiterer) Sensor zur Erfassung eines Durchmessers des Spritzenzylinders vorgesehen sein. Dieser Sensor kann wie der in der Druckschrift EP 1 329 232 B1 offenbarte Sensor zur Erfassung des Durchmessers des Spritzenzylinders ausgestaltet sein.

Für eine besonders umfassende Kolbenstangenüberwachung kann ein Ergebnis der erfindungsgemäßen Sensorik, also der Krafterfassungsvorrichtung und/oder der Wegerfassungsvorrichtung mit einem Ergebnis des (weiteren) Sensors abgeglichen werden. Bei einem Unterschied der beiden Ergebnisse oberhalb eines Grenzwertes ist insbesondere die Kolbenstange nicht korrekt am Kolben montiert. In diesem Fall kann das Signal, insbesondere Alarmsignal oder ein Hinweis im Display erzeugt werden.

### Kurzbeschreibung der Figuren

Figur 1 zeigt wesentliche Teile eines ersten Ausführungsbeispiels einer offenbarungsgemäßen Spritzenpumpe, und
Figur 2 zeigt wesentliche Teile eines zweiten Ausführungsbeispiels einer offenbarungsgemäßen Spritzenpumpe in einer geschnittenen Darstellung.

### Beschreibung bevorzugter Ausführungsbeispiele

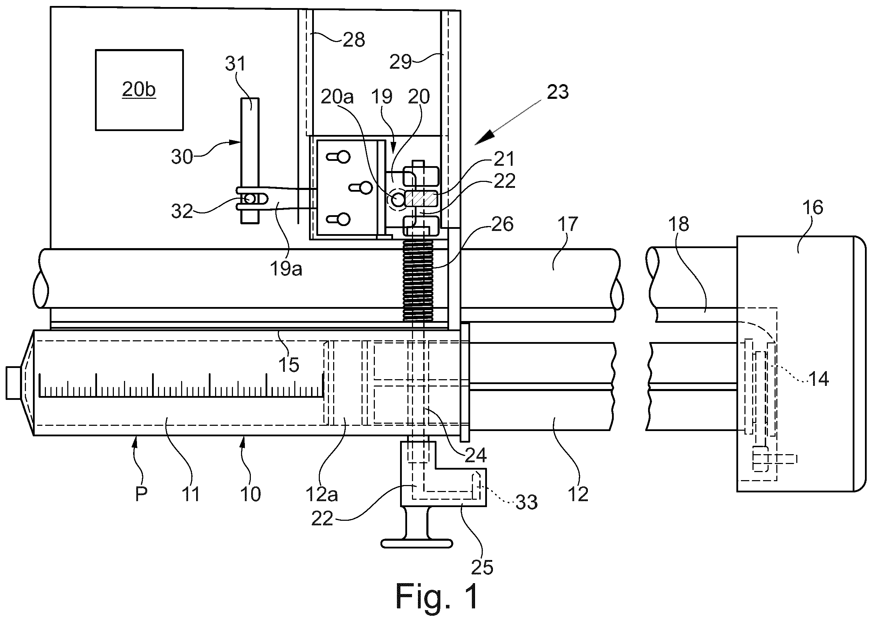

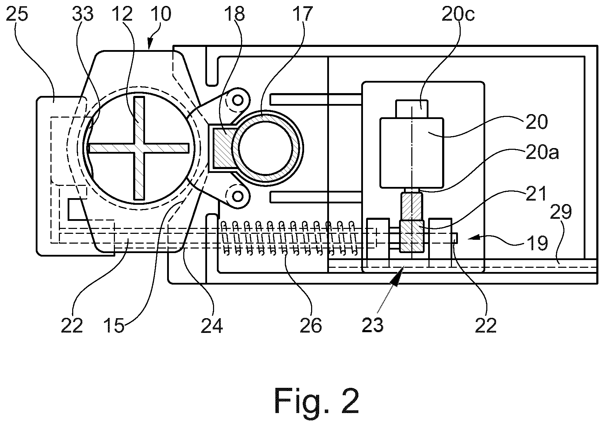

Die beiden Ausführungsbeispiele der Spritzenpumpe gemäß den Figuren 1 und 2 dienen zum Ausdrücken einer Spritze 10, welche einen Spritzenzylinder 11 und einen darin geführten Kolben 12a aufweist. Der Kolben 12a ist über ein (nicht näher gezeigtes) Schraubgewinde mit einer Spritzen-Kolbenstange 12 verbunden. Bei derartigen Spritzen (z.B. vorgefüllte Spritzen) besteht die Gefahr, dass die Kolbenstange 12 vom Anwender nicht korrekt in den Kolben 12a eingeschraubt wurde und damit ggf. keinen rechten Winkel zu diesem einnimmt oder beweglich zum Kolben ist und damit die Funktion der Spritze nicht mehr gewährleistet ist. Derartige und weitere (oben genannte) Fehler werden von beiden Ausführungsbeispielen der offenbarungsgemäßen Spritzenpumpe erkannt.

Die Spritze 10 ist in der dargestellten Sollposition in einen Aufnahmebereich P der Spritzenpumpe eingesetzt und in Anlage mit einem seitlichen Spritzenlager 15 der Spritzenpumpe. In diesem Zustand ist die Kolbenstange 12 aus dem Spritzenzylinder 11 herausgefahren und der Spritzenzylinder 11 ist mit Flüssigkeit gefüllt.

Die Kolbenstange 12 weist an ihrem vom Kolben 12a abgewandten Endabschnitt eine Spritzen-Kolbenplatte 14 auf. Zum Bewegen des Kolbens 12a über die Kolbenstange 12 und die Kolbenplatte 14 ist an der Spritzenpumpe ein linear bewegbarer Antriebskopf 16 (einer linearbeweglichen Baugruppe) vorgesehen, der an einer langgestreckten Antriebsstange 17 (der linearbeweglichen Baugruppe) angebracht ist. Der Antriebskopf 16 kann zunächst an die Kolbenplatte 14 herangefahren werden, um diese aufzunehmen und mit dem Antriebskopf 16 zu verriegeln. Die Kolbenplatte 14 kann dann, zusammen mit der Kolbenstange und dem Kolben, linear bewegt werden, um den Inhalt der Spritze 10 auszudrücken.

Auf der von einem Aufnahmebereich P abgewandten Seite der Antriebsstange 17 ist auf einem Schlitten 19 ein Elektromotor 20 eines Antriebs 23 einer Kolbenbremse befestigt. Der Schlitten 19 ist in Führungsschienen 28, 29 quer zum Spritzenzylinder 11 geführt. Der Elektromotor 20 wird über eine (nicht näher gezeigte) elektronische Motorsteuerung gesteuert und treibt über ein Schneckenrad seiner Motorwelle 20a ein ebenfalls auf dem Schlitten 19 gelagertes Zahnrad 21 mit Schrägverzahnung an. Das Zahnrad 21 ist mit einem Innengewinde versehen, die mit einem Außengewinde eines Endabschnitts einer Spindelstange 22 in Eingriff ist. Durch Drehen des Zahnrades 21 wird die Spindelstange 22, die gegen Mitdrehen gesichert ist, axial verschoben. Damit bildet die Spindelstange 22 einen Linearantrieb für das Bremselement 33 der Kolbenbremse.

Von dem Schlitten 19 erstreckt sich quer zum Spritzenzylinder 11 auch ein Rohr 24, durch das die Spindelstange 22 verläuft. An dem (in Figur 1 unteren) Endabschnitt des Rohres 24 ist ein Spritzenbügel 25 befestigt. Am Außenumfang des Rohres 24 sitzt eine schraubenförmige Feder 26, die an dem Spritzenlager 15 abgestützt ist und den Schlitten 19 von der Spritze 10 fortdrückt. Da das Rohr 24 mit dem Schlitten 19 verbunden ist, und da an dem Endabschnitt des Rohres 24 der Spritzenbügel 25 befestigt ist, wird dieser gegen die in dem Aufnahmebereich P befindliche Spritze 10 gedrückt und drückt damit die Spritze 10 gegen das Spritzenlager 15.

Die Spindelstange 22 der Kolbenbremse ragt in den Spritzenbügel 25 hinein und trägt das Bremselement 33 der Kolbenbremse, deren Schneide der Kolbenstange 12 radial zugewandt ist. Durch Betätigung des Elektromotors 20 wird das Bremselement 33 zwischen einer in Figur 1 gezeigten Rückzugsposition und einer in Figur 2 gezeigten aktiven Bremsposition radial angezogen und an die Kolbenstange 12 heranbewegt. In der Bremsposition greift das Bremselement 33 an der Kolbenstange 12 an, um Verschiebungen der Kolbenstange 12 in Bezug auf den Spritzenzylinder 11 zu blockieren. An der Antriebsstange 17 befindet sich ein Stützlager 18, gegen das sich die Kolbenplatte 14 bei Eingriff des Bremselements 33 abstützen kann.

Nach dem Loslassen des Spritzenbügels 25 bewirkt die Feder 26, dass der Spritzenbügel 25 den Spritzenzylinder 11 gegen das Spritzenlager 15 drückt. Der Spritzenzylinder 11 ist dann exakt positioniert.

Der Sensor 30 stellt anhand der Position, die der Schlitten 19 dann einnimmt, den Durchmesser der Spritzenzylinders 11 fest. Dazu wird ein Abgriff 32 eines Potentiometers 31 des Sensors 30 mit einem Schieber 19a, der mit dem Schlitten 19 verbunden ist, verstellt. Auf diese Weise gibt der elektrische Widerstand des Potentiometers 31 die Stellung des Schlittens 19 und somit auch die Stellung des Spritzenbügels 25 an. Das Signal des Sensors 30 gibt somit Aufschluss über den Durchmesser des Spritzenzylinders 11 bzw. die Spritzengröße, wenn der Spritzenbügel 25 radial am Spritzenzylinder 11 anliegt. Der Durchmesser der Spritzenzylinders 11 wird bei der Ermittlung der Vorschubgeschwindigkeit der Kolbenplatte 14 und damit des Kolbens 12a berücksichtigt.

Beim Loslassen des Spritzenbügels 25 wird der Elektromotor 20 in Funktion gesetzt, sodass das Bremselement 33 aus dem Spritzenbügel 25 herausgefahren und radial gegen die Kolbenstange 12 gedrückt wird. Dadurch wird die Kolbenstange 12 und damit der Kolben 12a in Bezug auf den Spritzenzylinder 11 fixiert.

Figur 1 zeigt das erste Ausführungsbeispiel der offenbarungsgemäßen Sensorik zur Kolbenstangenüberwachung und damit auch zur Spritzenüberwachung mittels der Kolbenbremse. Das erste Ausführungsbeispiel entspricht dem ersten Prinzip "Krafterfassung" und hat daher eine Erfassungsvorrichtung und/oder Ermittlungsvorrichtung, die dazu vorgesehen und ausgebildet ist, eine Kraft eines Bauteils des Antriebs 23 und damit eine Anwesenheit und/oder eine Position und/oder einen Durchmesser der Kolbenstange 12 zu erfassen und/oder zu ermitteln.

Genauer gesagt weist die (nicht näher gezeigte) elektronische Motorsteuerung eine (symbolisch dargestellte) Motorstromerfassungsvorrichtung 20b auf, mit der ein Drehmomentsensor für die Motorwelle 20a gebildet ist. Wenn das Bremselement 33 in Anlage mit der korrekt eingeschraubten und am Stützlager 18 abgestützten Kolbenstange 12 gerät, steigt der Motorstrom über einen Grenzwert an, und die Motorstromerfassungsvorrichtung 20b erkennt so die radiale Erstreckung bzw. Dicke der Kolbenstange 12. Dieses Ergebnis der radialen Erstreckung bzw. Dicke wird einem Plausibilitätstest unterzogen.

Wenn mittels der Motorstromerfassungsvorrichtung 20b auch ein Motorstromanstieg erfasst wird, kann auch erkannt werden, wenn die Kolbenstange 12 zu wenig mechanischen Wiederstrand bietet. Damit wird insbesondere erkannt, wenn die Kolbenstange 12 nicht stabil ist und damit nicht korrekt in den Kolben 12a eingeschraubt ist.

Figur 2 zeigt das zweite Ausführungsbeispiel der offenbarungsgemäßen Sensorik zur Kolbenstangenüberwachung und damit auch zur Spritzenüberwachung mittels der Kolbenbremse. Das zweite Ausführungsbeispiel entspricht dem zweiten Prinzip "Wegerfassung" und hat daher eine Erfassungsvorrichtung und/oder Ermittlungsvorrichtung, die dazu vorgesehen und ausgebildet ist, einen Weg eines Bauteils des Antriebs 23 und damit eine Anwesenheit und/oder eine Position und/oder einen Durchmesser der Kolbenstange 12 zu erfassen und/oder zu ermitteln.

Genauer gesagt ist an einem vom Schneckenrad abgewandten Endabschnitt der Motorwelle 20a des Elektromotors 20 eine Umdrehungserfassungsvorrichtung 20c angeordnet. Diese ist von einem rotatorischen Potentiometer gebildet. Die Umdrehungserfassungsvorrichtung 20c ist am Gehäuse des Elektromotors 20 befestigt und ist in Wirkverbindung mit der Motorwelle 20a. Sie erfasst die Anzahl der Umdrehungen der Motorwelle 20a und kann damit auf die Position der Bremselements 33, genauer gesagt auf dessen Abstand zum Stützlager 18 schließen. Die Umdrehungserfassungsvorrichtung 20c erkennt so die radiale Erstreckung bzw. Dicke der Kolbenstange 12. Dieses Ergebnis der radialen Erstreckung bzw. Dicke wird einem Plausibilitätstest unterzogen.

Nach dem Plausibilitätstest kann der Motorstrom des Elektromotors 20 abgeschaltet werden. Dann wird automatisch die Antriebsstange 17 derart eingezogen, dass der Antriebskopf 16 an die Kolbenplatte 14 heranfährt. Wenn ein Kolbensensor 40 des Antriebskopfes 16 das Anliegen der Kolbenplatte 14 feststellt, wird diese im Antriebskopf 16 verriegelt.

Dann wird der Elektromotor 20 der Kolbenbremse in Gegenrichtung betätigt, um das Bremselement 33 wieder von der Kolbenstange 12 zu entfernen. Daraufhin kann der Normalbetrieb der Spritzenpumpe beginnen und dazu der Antriebskopf 16 über die Antriebsstange 17 zum Ausdrücken der Spritze 10 eingezogen werden.

Es können auch bei Prinzipien der offenbarungsgemäßen Sensorik in der Kolbenbremse kombiniert sein. Bei einem Ausführungsbeispiel wäre dann die Motorstromerfassungsvorrichtung 20b aus Figur 1 mit der Umdrehungserfassungsvorrichtung 20c aus Figur 2 kombiniert.

Statt der gezeigten Zustellbewegungsrichtung und Ausrichtung des Bremselements 33 rechtwinklig zur Kolbenstange 12 kann auch eine Zustellbewegungsrichtung und Ausrichtung des Bremselements 33 schräg zur Kolbenstange 12 vorgesehen sein. Insbesondere bietet sich dabei eine Anstellung schräg gegen die Einschubrichtung an, mit der die Kolbenstange 12 später in den Spritzenzylinder 11 eingeschoben wird. Damit kann z.B. auch eine etwas abgestumpfte Schneide des Bremselements 33 und/oder eine schwächere Kraft des Antriebs 23 toleriert werden.

Statt der gezeigten linearen Zustellbewegung der Bremselements 33 kann diese auch durch eine bogenförmige Schwenkbewegung zugestellt werden, solange zumindest eine Bewegungskomponente in radialer Richtung zur Kolbenstange 12 und zur Spritzenlängsachse gegeben ist.

Statt der gezeigten Anordnung des Bremselements 33 an der von der Antriebsstange 17 abgewandten Seite der Spritze 10 (quasi an der distalen Seite der Spritzenpumpe) kann das Bremselement 33 selbstverständlich auch an einer anderen Seite am Umfang des Kolbenstange 12 angeordnet sein, insbesondere an der Seite, an der auch die Antriebsstange 17 angeordnet ist (quasi an der proximalen Seite der Spritzenpumpe).

Statt der gezeigten Integration des Bremselements 33 in den Spritzenbügel 25 kann das Bremselement 33 auch beabstandet vom Spritzenbügel 25 und/oder mechanisch von diesem entkoppelt sein.

Statt des gezeigten einen Bremselements 33 können auch mehrere Bremselemente 33 am Außenumfang der Kolbenstange 12 verteilt sein, die entsprechend von mehreren Seiten gleichzeitig an die Kolbenstange 12 heranbewegt werden.

Statt mittels der gezeigten Schneide am Bremselement 33 kann das Bremselement 33 die Kolbenstange 12 auch mit einer erhöhten Kraft und per Reibschluss halten.

## Patentansprüche

1. Spritzenpumpe mit einem Aufnahmebereich (P) für eine Spritze (10), wobei ein Antriebskopf (16) der Spritzenpumpe linear in den Aufnahmebereich (P) bewegbar ist, und wobei mindestens ein Bremselement (33) einer Kolbenbremse zu einer Kolbenstange (12) der Spritze (10) bewegbar und mit dieser in Anlage bringbar ist, um die Kolbenstange (12) kraftschlüssig und/oder formschlüssig festzulegen, **dadurch gekennzeichnet, dass** ein Antrieb (23) der Kolbenbremse eine Erfassungsvorrichtung und/oder Ermittlungsvorrichtung aufweist, die dazu vorgesehen und ausgebildet ist, eine Kraft und/oder einen Weg eines Bauteils des Antriebs (23) und damit eine Anwesenheit und/oder eine Position und/oder einen Durchmesser der Kolbenstange (12) zu erfassen und/oder zu ermitteln.

2. Spritzenpumpe nach Anspruch 1, wobei der Antrieb (23) einen Elektromotor (20) aufweist.

3. Spritzenpumpe nach einem der vorhergehenden Ansprüche, wobei von der Erfassungsvorrichtung und/oder Ermittlungsvorrichtung ein Kraftanstieg erfassbar und/oder ermittelbar ist, und wobei erfassbar und/oder ermittelbar ist, wenn der Kraftanstieg unterhalb eines Grenzwertes ist.

4. Spritzenpumpe nach einem der vorhergehenden Ansprüche, wobei die Erfassungsvorrichtung und/oder Ermittlungsvorrichtung vorgesehen und ausgebildet ist, ein Drehmoment zu erfassen und/oder zu ermitteln.

5. Spritzenpumpe nach den Ansprüchen 2 und 4, wobei die Erfassungsvorrichtung eine Motorstromerfassungsvorrichtung (20b) ist, mit der der Motorstrom des Elektromotors (20) erfassbar ist.

6. Spritzenpumpe nach Anspruch 5, wobei mittels der Motorstromerfassungsvorrichtung (20b) ein Motorstromanstieg erfassbar ist, und wobei erfassbar ist, wenn der Motorstromanstieg geringer als ein Grenzwert ist.

7. Spritzenpumpe nach einem der vorhergehenden Ansprüche, wobei die Erfassungsvorrichtung eine Umdrehungserfassungsvorrichtung (20c) ist, die mit einem rotierenden Bauteil des Antriebs (23) in Wirkverbindung ist.

8. Spritzenpumpe nach den Ansprüchen 2 und 7, wobei das rotierende Bauteil eine Motorwelle (20a) oder ein Gehäuse des Elektromotors (20) ist.

9. Spritzenpumpe nach Anspruch 1 oder 7 oder 8, wobei die Erfassungsvorrichtung ein Potentiometer ist.

10. Spritzenpumpe nach einem der vorhergehenden Ansprüche mit mehreren Bremselementen (33), die am Außenumfang der Kolbenstange (12) verteilt sind.

11. Spritzenpumpe nach einem der vorhergehenden Ansprüche mit einem Sensor (30) zur Ermittlung eines Durchmessers eines Spritzenzylinders (11) der Spritze (10).

12. Spritzenpumpe nach Anspruch 11, wobei ein von der Erfassungsvorrichtung erfasster Weg des Bauteils mit einem vom Sensor (30) ermittelten Durchmesser abgleichbar ist, und wobei erfassbar ist, wenn ein Unterschied der beiden Ergebnisse größer als ein Grenzwert ist.

## Claims

1. A syringe pump with a receiving region (P) for a syringe (10), wherein a drive head (16) of the syringe pump can be moved linearly into the receiving region (P), and wherein at least one brake element (33) of a piston brake is movable to a piston rod (12) of the syringe (10) and can be brought into contact therewith in order to fix the piston rod (12) with force-fit and/or form-fit,
**characterized in that** a drive (23) of the piston brake has a detection device and/or ascertaining device which is provided and configured to detect and/or ascertain a force and/or a travel of a component of the drive (23) and thus a presence and/or a position and/or a diameter of the piston rod (12).

2. The syringe pump of claim 1, wherein the drive (23) comprises an electric motor (20).

3. The syringe pump according to one of the preceding claims, wherein a force increase is detectable and/or ascertainable by the detecting device and/or ascertaining device, and wherein it is detectable and/or ascertainable when the force increase is below a threshold value.

4. The syringe pump according to one of the preceding claims, wherein the detection device and/or ascertaining device is provided and configured to detect and/or ascertain a torque.

5. The syringe pump according to claims 2 and 4, wherein the detection device is a motor current detection device (20b) with which the motor current of the electric motor (20) is detectable.

6. The syringe pump according to claim 5, wherein a motor current increase is detectable via the motor current detection device (20b), and wherein it is detectable when the motor current increase is less than a threshold value.

7. The syringe pump according to one of the preceding claims, wherein the detection device is a revolution detection device (20c) and is on operative connection with a rotating component of the drive (23).

8. The syringe pump according to claims 2 and 7, wherein the rotating component is a motor shaft (20a) or a housing of the electric motor (20).

9. The syringe pump according to claim 1 or 7 or 8, wherein the detection device is a potentiometer.

10. The syringe pump according to one of the preceding claims, comprising multiple brake elements (33) distributed around the outer circumference of the piston rod (12).

11. The syringe pump according to one of the preceding claims, comprising a sensor (30) for detecting a diameter of a syringe cylinder (11) of the syringe (10).

12. The syringe pump according to claim 11, wherein a travel of the component detected by the detection device is matchable with a diameter ascertained by the sensor (30), and wherein it is detectable when a difference of the two results is greater than a threshold value.

## Revendications

1. Pompe à seringue dotée d'une zone de réception (P) destinée à une seringue (10), dans laquelle une tête d'entraînement (16) de la pompe à seringue peut être déplacée linéairement dans la zone de réception (P), et dans laquelle au moins un élément de freinage (33) d'un frein à piston peut être déplacé vers une tige de piston (12) de la seringue (10) et peut être mis en contact avec celle-ci afin de fixer la tige de piston (12) par liaison de force et/ou par liaison de forme, **caractérisée en ce que** un entraînement (23) du frein à piston présente un dispositif de détection et/ou un dispositif de détermination, qui est(sont) prévu(s) et conçu(s) pour détecter et/ou déterminer une force et/ou une course d'un composant de l'entraînement (23) et donc la présence et/ou la position et/ou le diamètre de la tige de piston (12).

2. Pompe à seringue selon la revendication 1, dans laquelle l'entraînement (23) présente un moteur électrique (20).

3. Pompe à seringue selon l'une quelconque des revendications précédentes, dans laquelle une augmentation de force peut être détectée et/ou déterminée par le dispositif de détection et/ou le dispositif de détermination, et dans laquelle elle peut être détectée et/ou déterminée, si l'augmentation de force est inférieure à une valeur seuil.

4. Pompe à seringue selon l'une quelconque des revendications précédentes, dans laquelle le dispositif de détection et/ou le dispositif de détermination est(sont) prévu(s) et conçu(s) pour détecter et/ou déterminer un couple de rotation.

5. Pompe à seringue selon les revendications 2 et 4, dans laquelle le dispositif de détection est un dispositif de détection de courant du moteur (20b), grâce auquel le courant du moteur du moteur électrique (20) peut être détecté.

6. Pompe à seringue selon la revendication 5, dans laquelle une augmentation de courant du moteur peut être détectée au moyen du dispositif de détection de courant du moteur (20b), et dans laquelle elle peut être détectée, si l'augmentation de courant du moteur est inférieure à une valeur seuil.

7. Pompe à seringue selon l'une quelconque des revendications précédentes, dans laquelle le dispositif de détection est un dispositif de détection de rotation (20c), qui est en liaison fonctionnelle avec un composant rotatif de l'entraînement (23).

8. Pompe à seringue selon les revendications 2 et 7, dans laquelle le composant rotatif est un arbre de moteur (20a) ou un logement du moteur électrique (20).

9. Pompe à seringue selon la revendication 1 ou 7 ou 8, dans laquelle le dispositif de détection est un potentiomètre.

10. Pompe à seringue selon l'une quelconque des revendications précédentes comprenant plusieurs éléments de freinage (33), qui sont répartis sur une périphérie extérieure de la tige de piston (12).

11. Pompe à seringue selon l'une quelconque des revendications précédentes comprenant un capteur (30) pour la détermination d'un diamètre d'un cylindre de seringue (11) de la seringue (10).

12. Pompe à seringue selon la revendication 11, dans laquelle une course du composant détectée par le dispositif de détection peut être comparée à un diamètre déterminé par le capteur (30), et dans laquelle elle peut être détectée, si une différence entre les deux résultats est supérieure à une valeur seuil.
